# EUROPEAN PATENT APPLICATION

(11) **EP 2 093 567 A1**
(43) Date of publication of application: **26.08.2009**
(21) Application number: 08380053.2
(22) Date of filing: 21.02.2008
(51) Int. Cl.: G01N 33/574, C12Q 1/68

(54) **Brca1 mRNA expression levels predict survival in breast cancer patients treated with neoadjuvant chemotherapy**

(71) Applicant: Pangaea Biotech, S.A., 08028 Barcelona (ES)
(72) Inventor: Tarón Roca, Miguel, 08916 Badalona (ES); Rosell Costa, Rafael, 08916 Badalona (ES)
(74) Representative: ABG Patentes, S.L.

(57) **Abstract**

The invention relates to methods for predicting the clinical outcome of a patient which suffers from breast cancer based on the expression levels of BRCA1, wherein low BRCA1 expression levels are indicative of a good prognosis. Moreover, the invention relates to methods for predicting the response to a neoadjuvant therapy based on a combination of an anti-metabolite, an intercalating agent and an alkylating agent of a patient which suffers from breast cancer based on the expression levels of BRCA1.

## Description

### FIELD OF THE INVENTION

The present invention relates to the field of diagnostics and, in particular, to a method for selecting an individual neoadjuvant chemotherapy and for predicting the survival of breast cancer patients, based on the expression level of the BRCA1 gene in a sample from said patient.

### BACKGROUND OF THE INVENTION

Worldwide, breast cancer is the second most common type of cancer (10.4%; after lung cancer) and the fifth most common cause of cancer death (after lung cancer, stomach cancer, liver cancer, and colon cancer). Among women worldwide, breast cancer is the most common cause of cancer death. In 2005, breast cancer caused 502,000 deaths worldwide (7% of cancer deaths; almost 1% of all deaths). The number of cases worldwide has significantly increased since the 1970s, a phenomenon partly blamed on modem lifestyles in the Western world. North American women have the highest incidence of breast cancer in the world.

Because the breast is composed of identical tissues in males and females, breast cancer also occurs in males. Incidences of breast cancer in men are approximately 100 times less common than in women, but men with breast cancer are considered to have the same statistical survival rates as women.

Breast cancer is staged according to the TNM system. Prognosis is closely linked to results of staging, and staging is also used to allocate patients to treatments both in clinical trials and clinical practice. The information for staging is as follows:
- TX: Primary tumor cannot be assessed. T0: No evidence of tumor. Tis: Carcinoma in situ, no invasion T1: Tumor is 2 cm or less T2: Tumor is more than 2 cm but not more than 5 cm T3: Tumor is more than 5 cm T4: Tumor of any size growing into the chest wall or skin, or inflammatory breast cancer
- NX: Nearby lymph nodes cannot be assessed. N0: Cancer has not spread to regional lymph nodes. N1: Cancer has spread to 1 to 3 axillary or one internal mammary lymph node N2: Cancer has spread to 4 to 9 axillary lymph nodes or multiple internal mammary lymph nodes N3: One of the following applies:
   o Cancer has spread to 10 or more axillary lymph nodes, or Cancer has spread to the lymph nodes under the clavicle (collar bone), or Cancer has spread to the lymph nodes above the clavicle, or Cancer involves axillary lymph nodes and has enlarged the internal mammary lymph nodes, or Cancer involves 4 or more axillary lymph nodes, and tiny amounts of cancer are found in internal mammary lymph nodes on sentinel lymph node biopsy.
   - MX: Presence of distant spread (metastasis) cannot be assessed. M0: No distant spread. M1: Spread to distant organs, not including the supraclavicular lymph node, has occurred.

The mainstay of breast cancer treatment is surgery when the tumor is localized, with possible adjuvant hormonal therapy (with tamoxifen or an aromatase inhibitor), chemotherapy, and/or radiotherapy. At present, the treatment recommendations after surgery (adjuvant therapy) follow a pattern. This pattern is subject to change, as every two years, a worldwide conference takes place in St. Gallen, Switzerland, to discuss the actual results of worldwide multi-center studies.

On the other hand, neoadjuvant chemotherapy, an adjunctive therapy given before a definitive treatment, is an essential component of modem multidisciplinary cancer therapy. Although neoadjuvant or induction therapy does not contribute the most to the treatment outcome, it may improve the result substantially. For example, neoadjuvant therapy allows patients with large breast cancer to undergo breast-conserving surgery. It enables patients with locally advanced laryngeal cancer to have their vocal function preserved. Many patients with rectal cancer can avoid permanent colostomy after undergoing this approach. In addition, in certain cancers, neoadjuvant therapy may improve long-term survival. Mouret-Reynier et al. (Clin Breast Cancer. 2004 Oct;5 (4):303-7) investigated the efficacy of FEC as neoadjuvant chemotherapy in women with stage I-III primary operable breast cancer concluding that it was effective and well tolerated in patients with early-stage operable breast cancer.

During the past 30 years medical oncologists have focused to optimise the outcome of cancer patients and it is just now that the new technologies available are allowing to investigate polymorphisms, gene expression levels and gene mutations aimed to predict the impact of a given therapy in different groups of cancer patients to tailor chemotherapy. Representative examples include the relation between the TS mRNA expression and the response and the survival with antifolates (see EP 1 381 691), beta tubulin III mRNA levels and response to tubulin interacting agents, PTEN methylation and resistance to CPT-11 and STAT3 over expression and resistance to EGF interacting agents. PCR tests like Oncotype DX or microarray tests like MammaPrint can predict breast cancer recurrence risk based on gene expression. In February 2007, the MammaPrint test became the first breast cancer predictor to win formal approval from the Food and Drug Administration. This is a new gene test to help predict whether women with early-stage breast cancer will relapse in 5 or 10 years, this could help influence how aggressively the initial tumor is treated.

Breast Cancer 1 (BRCA1) plays a crucial role in DNA repair, and decreased BRCA1 mRNA expression has been observed in both sporadic and hereditary breast cancers (Kennedy RD, et al. (2002) Lancet, 360, 1007-1014). These patients can respond to DNA damage-based chemotherapy but not to antimicrotubule drugs. In addition, DNA damage-based chemotherapy confers a significant survival advantage to BRCA1 mutation carriers compared to non-mutation carriers. Also ovarian cancer patients with low levels of BRCA1 mRNA have improved survival following platinum-based chemotherapy compared to patients with high levels of BRCA1 mRNA (Quinn et al, Clin Cancer Res. 2007 Dec 15;13(24):7413-20).

BRCA1 is implicated in transcription-coupled nucleotide excision repair (TC-NER), and modulation of its expression leads to modification of TC-NER and hence to radio- and chemoresistance. Upregulation of BRCA1 expression led to increased cisplatin resistance in the SKOV-3 human ovarian cancer cell line (Husain A, et al. Cancer Res. 1998 Mar 15;58(6):1120-3) and restoration of BRCA1 in the BRCA1-negative HCC1937 human breast cancer cell line restored radioresistance. BRCA1 is also involved in homologous recombination repair (HRR) and non-homologous end joining in response to DNA damage. In addition, it is a component of a large DNA repair complex termed the BRCA1-associated genome surveillance complex, which contains a number of mismatch repair proteins, indicating a potential role for BRCA1 in mismatch repair. BRCA1 may also be a regulator of mitotic spindle assembly, as BRCA1 and β-tubulin colocalize to the microtubules of the mitotic spindle and to the centrosomes. Finally, enhanced BRCA1 expression has been linked to apoptosis through the c-Jun N-terminal kinase pathway, which is activated by cisplatin-induced DNA damage; inhibition of this pathway increased cisplatin sensitivity in cell lines. Decreased BRCA1 mRNA expression in a breast cancer cell line, as determined by real-time quantitative polymerase chain reaction (RT-QPCR), led to greater sensitivity to cisplatin and etoposide but to greater resistance to the microtubule-interfering agents paclitaxel and vincristine (Lafarge S, et al. (2001) Oncogene, 20, 6597-6606). Recently, reconstitution of wild-type BRCA1 into the BRCA1-negative HCC1937 breast cancer cell line resulted in a 20-fold increase in cisplatin resistance and, in contrast, in a 1000-10,000-fold increase in sensitivity to antimicrotubule drugs (paclitaxel and vinorelbine).

Mouse models carrying conditional disruption of BRCA1 were highly sensitive to doxorubicin and gamma irradiation but resistant to tamoxifen, providing additional evidence for differential chemosensitivity linked to BRCA1 expression. When BRCA1 expression was examined by semi-quantitative PCR in women with sporadic breast cancer, lower BRCA1 mRNA levels (bottom quartile) were associated with a higher frequency of distant metastases (Seery LT, et al. (1999) Int. J. Cancer (Pred. Oncol.), 84, 258-262.

Despite the wealth of data in cell lines and mouse models, only one small study has examined the correlation of BRCA1 and BRCA2 mRNA expression with response to chemotherapy in the clinical setting (Egawa C., (2001) Int. J. Cancer (Pred. Oncol.), 95, 255-259). Among 25 women with docetaxel-treated locally advanced or metastatic breast cancer, only BRCA2 mRNA levels were significantly lower in responders than in non-responders, though a slight difference was also observed for BRCA1.

Martin-Richard et al (Oncology, 2004; 66: 388-94) describes the value of topoisomerase IIalpha (Topo II) in predicting the clinical response to anthracycline-based neoadjuvant chemotherapy in breast cancers and the potential changes in Topo II after chemotherapy. The results show that Topo II was overexpressed in 31 % of tumors before treatment, and this overexpression was significantly associated with clinical response. Kandioler-Eckersberger D. et al (Clin Cancer Res. 2000; 6:50-6) describes the value of p53 to predict the cytotoxic effect of FEC (fluorouracil, epirubicin and cyclophosphamide) and microtubule stabilizing (paclitaxel) chemotherapies regimens in patients with advanced breast cancer. The results show that response to a combination of FEC was directly related to normal p53 and tumor cell apoptosis in breast cancer patients. Knoop (J Clin Oncol., 2005; 23:7483-90) describes that patients with *TOP2A* amplification had an increased recurrence-free and overall survival, respectively, if treated with CEF (cyclophosphamide, epirubicin, and fluorouracil) compared with CMF (cyclophosphamide, methotrexate, and fluorouracil) chemotherapies, and that patients with *TOP2A* deletions had an almost identical hazard ratio.

It is an object of the present invention to provide predictors of response to chemotherapy, in particular to neoadjuvant therapy, which can be a valuable clinical tool for use in the selection of optimal treatment modes, in particular for patients suffering from breast cancer.

### SUMMARY OF THE INVENTION

The present invention provides a tool for use in predicting differential chemosensitivity and tailoring neoadjuvant chemotherapy in breast cancer.

Inventors have surprisingly found that a neoadjuvant therapy based on a combination of an anti-metabolite, an intercalating agent and an alkylating agent improved survival in patients suffering from breast cancer with low expression levels of BRCA1.

Thus, in a first aspect, the present invention refers to a method for selecting an individual neoadjuvant therapy for a patient suffering from breast cancer which comprises determining the expression levels of BRCA1 gene in a sample from said patient, wherein if expression levels of BRCA1 gene are low when compared with reference values then, the patient is a good candidate for a neoadjuvant therapy based on a combination of an anti-metabolite, an intercalating agent and an alkylating agent.

In a second aspect, the invention refers to a method for determining the clinical response of a patient suffering from breast cancer to neoadjuvant therapy based on a combination of an anti-metabolite, an intercalating agent and an alkylating agent which comprises determining BRCA1 gene expression levels in a sample from said patient, wherein if the expression levels of BRCA1 gene are low when compared with reference values, then it is indicative of a good clinical response of said patient to said therapy.

In a further aspect, the invention relates to a method for evaluating the predisposition of a patient suffering from breast cancer to respond to a neoadjuvant therapy based on a combination of an anti-metabolite, an intercalating agent and an alkylating agent which comprises determining the expression levels of BRCA1 gene in a sample from said patient, wherein if the expression levels of BRCA1 gene are low, then it is indicative of favourable predisposition of said patient to respond to a neoadjuvant therapy based on a combination of an anti-metabolite, an intercalating agent and an alkylating agent.

In another aspect, the invention refers to a combination of an anti-metabolite, an intercalating agent and an alkylating agent as a neoadjuvant therapy for the treatment of breast cancer in a patient suffering from breast cancer wherein said patient presents low expression levels of the BRCA1 gene.

In another aspect, the invention relates to a method for classifying patients suffering from breast cancer comprising determining:
i) the expression levels of BRCA1 gene; and
ii) progesterone receptor expression;
iii) classifying the patients in four groups according to the results of step i) and ii) defined as
   - low expression levels of BRCA1 gene and positive progesterone receptor expression;
   - low expression levels of BRCA1 and negative progesterone receptor expression;
   - high expression levels of BRCA1 gene and positive progesterone receptor expression; and
   - high expression levels of BRCA1 and negative progesterone receptor expression.

### BRIEF DESCRIPTION OF THE FIGURES

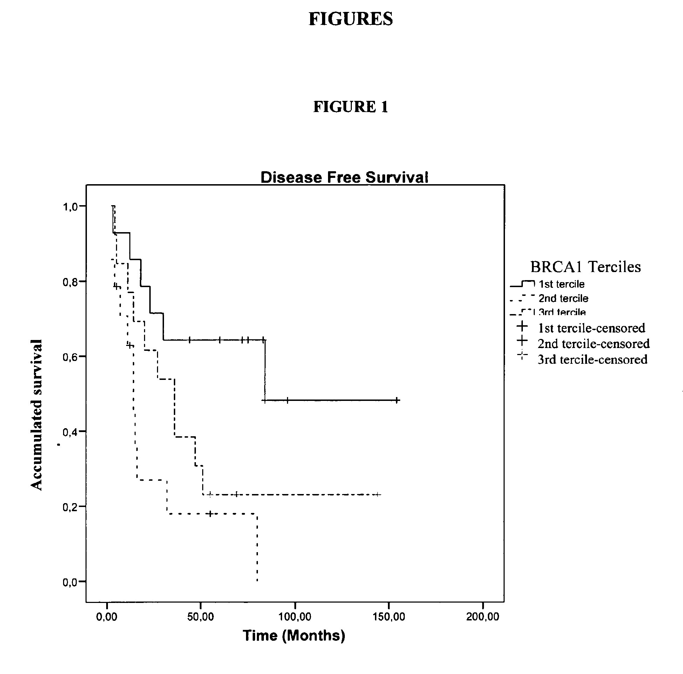

**Figure 1** shows a Kaplan-Meier survival curve representing disease free survival (DFS) for BRCA1 by terciles. Time is represented in months. On the plot, small vertical tick-marks indicate losses, where patient data has been censored. The term "censored" indicates losses from the sample before the final outcome is observed.

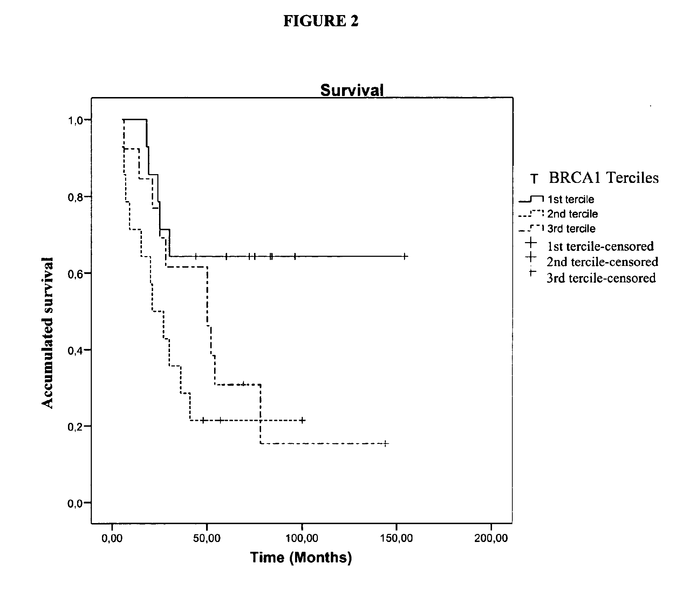

**Figure 2** shows a Kaplan-Meier survival curve representing median survival for BRCA1 by terciles. Time is represented in months. On the plot, small vertical tick-marks indicate losses, where patient data has been censored. The term "censored" indicates losses from the sample before the final outcome is observed.

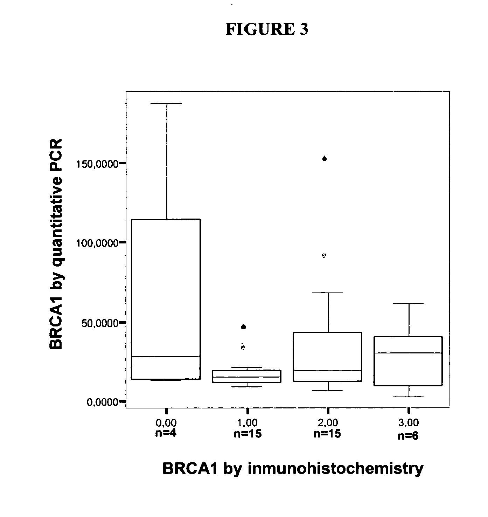

**Figure 3** is a graph representing correlation between BRCA1 protein expression measured by immunohistochemistry (values 0, 1, 2, and 3) and BRCA1 mRNA sputum, cerebral spinal fluid (CSF), tears, mucus, sweat, milk, brain extracts and the like). In a particular embodiment, said sample is a tumour tissue sample or portion thereof. In a more particular embodiment, said tumor tissue sample is a breast tumor tissue sample from a patient suffering from breast cancer. Said sample can be obtained by conventional methods, e.g., biopsy, by using methods well known to those of ordinary skill in the related medical arts. Methods for obtaining the sample from the biopsy include gross apportioning of a mass, or microdissection or other art-known cell-separation methods. Tumour cells can additionally be obtained from fine needle aspiration cytology. In order to simplify conservation and handling of the samples, these can be formalin-fixed and paraffin-embedded or first frozen and then embedded in a cryosolidifiable medium, such as OCT-Compound, through immersion in a highly cryogenic medium that allows for rapid freeze.

The method of the invention requires determining the expression levels of the BRCA1 gene. In a preferred embodiment, the determination of the expression levels of the BRCA1 gene can be carried out by measuring the expression levels of the mRNA encoded by the BRCA1 gene. For this purpose, the biological sample may be treated to physically or mechanically disrupt tissue or cell structure, to release intracellular components into an aqueous or organic solution to prepare nucleic acids for further analysis. The nucleic acids are extracted from the sample by procedures known to the skilled person and commercially available. RNA is then extracted from frozen or fresh samples by any of the methods typical in the art, for example, Sambrook, Fischer and Maniatis, Molecular Cloning, a laboratory manual, (2nd ed.), Cold Spring Harbor Laboratory Press, New York, (1989). Preferably, care is taken to avoid degradation of the RNA during the extraction process.

In a particular embodiment, the expression level is determined using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene, for example. Deparaffinized samples can be rehydrated with an aqueous solution of a lower alcohol. Suitable lower alcohols, for example include, methanol, ethanol, propanols, and butanols. Deparaffinized samples may be rehydrated with successive washes with lower alcoholic solutions of decreasing concentration, for example. Alternatively, the sample sputum, cerebral spinal fluid (CSF), tears, mucus, sweat, milk, brain extracts and the like). In a particular embodiment, said sample is a tumour tissue sample or portion thereof. In a more particular embodiment, said tumor tissue sample is a breast tumor tissue sample from a patient suffering from breast cancer. Said sample can be obtained by conventional methods, e.g., biopsy, by using methods well known to those of ordinary skill in the related medical arts. Methods for obtaining the sample from the biopsy include gross apportioning of a mass, or microdissection or other art-known cell-separation methods. Tumour cells can additionally be obtained from fine needle aspiration cytology. In order to simplify conservation and handling of the samples, these can be formalin-fixed and paraffin-embedded or first frozen and then embedded in a cryosolidifiable medium, such as OCT-Compound, through immersion in a highly cryogenic medium that allows for rapid freeze.

The method of the invention requires determining the expression levels of the BRCA1 gene. In a preferred embodiment, the determination of the expression levels of the BRCA1 gene can be carried out by measuring the expression levels of the mRNA encoded by the BRCA1 gene. For this purpose, the biological sample may be treated to physically or mechanically disrupt tissue or cell structure, to release intracellular components into an aqueous or organic solution to prepare nucleic acids for further analysis. The nucleic acids are extracted from the sample by procedures known to the skilled person and commercially available. RNA is then extracted from frozen or fresh samples by any of the methods typical in the art, for example, Sambrook, Fischer and Maniatis, Molecular Cloning, a laboratory manual, (2nd ed.), Cold Spring Harbor Laboratory Press, New York, (1989). Preferably, care is taken to avoid degradation of the RNA during the extraction process.

In a particular embodiment, the expression level is determined using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene, for example. Deparaffinized samples can be rehydrated with an aqueous solution of a lower alcohol. Suitable lower alcohols, for example include, methanol, ethanol, propanols, and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample.

While all techniques of gene expression profiling (RT-PCR, SAGE, or TaqMan) are suitable for use in performing the foregoing aspects of the invention, the gene mRNA expression levels are often determined by reverse transcription polymerase chain reaction (RT-PCR). The detection can be carried out in individual samples or in tissue microarrays.

In order to normalize the values of mRNA expression among the different samples, it is possible to compare the expression levels of the mRNA of interest in the test samples with the expression of a control RNA. A "Control RNA" as used herein, relates to a RNA whose expression levels do not change or change only in limited amounts in tumor cells with respect to non-tumorigenic cells. Preferably, the control RNA is mRNA derived from housekeeping genes and which code for proteins which are constitutively expressed and carry out essential cellular functions. Preferred housekeeping genes for use in the present invention include β-2-microglobulin, ubiquitin, 18-S ribosomal protein, cyclophilin, GAPDH and actin. In a preferred embodiment, the control RNA is BETA-actin mRNA. In one embodiment relative gene expression quantification is calculated according to the comparative Ct method using β-actin as an endogenous control and commercial RNA controls as calibrators. Final results, are determined according to the formula 2-(ΔCt sample-ΔCt calibrator), where ΔCT values of the calibrator and sample are determined by subtracting the CT value of the target gene from the value of the β-actin gene.

The determination of the level of expression of the BRCA1 gene needs to be correlated with the reference values which correspond to the median value of expression levels of BRCA1 measured in a collection of tumor tissue in biopsy samples from cancer patients, previous to the neoadjuvant chemotherapeutic treatment. Once this median value is established, the level of this marker expressed in tumor tissues from patients can be compared with this median value, and thus be assigned a level of "low," "normal" or "high". The collection of samples from which the reference level is derived will preferably be constituted from patient suffering from the same type of cancer. For example, the one described in the examples which is statistically representative was constituted with 41 samples from breast cancer patients. In any case it can contain a different number of samples. The use of a reference value used for determining whether the expression of a gene sample is "increased" or "decreased" corresponds to the median value of expression levels of BRCA1 measured in a RNA sample obtained by pooling equal amounts of RNA from each of the tumour samples obtained by biopsy from cancer patients previous to the neoadjuvant chemotherapeutic treatment. Once this median value is established, the level of this marker expressed in tumours tissues from patients can be compared with this median value, and thus be assigned a level of "increased" or "decreased". Due to inter-subject variability (e.g. aspects relating to age, race, etc.) it is very difficult (if not practically impossible) to establish absolute reference values for BRCA1. Thus, in a particular embodiment, the reference values for "increased" or "decreased" BRCA1 expression are determined by calculating percentiles by conventional means involving the testing of a group of samples isolated from normal subjects (i.e. people with no diagnosis of breast cancer) for the expression levels of the BRCA1 gene. The "increased" levels can then be assigned, preferably, to samples wherein expression levels for the BRCA1 genes are equal to or in excels of percentile 50 in the normal population, including, for example, expression levels equal to or in excess to percentile 60 in the normal population, equal to or in excess to percentile 70 in the normal population, equal to or in excess to percentile 80 in the normal population, equal to or in excess to percentile 90 in the normal population, and equal to or in excess to percentile 95 in the normal population.

In a preferred embodiment BRCA1 expression values are divided into terciles. As an example, real-time quantitative PCR was used to determine BRCA1 mRNA levels in 41 tumor biopsies from breast cancer patients who had received neoadjuvant FEC chemotherapy, and divided the gene expression values into terciles. When results were correlated with outcome (DFS and MS), it was observed that patients with BRCA1 levels in the bottom tercile (tercile 1) had a significantly decreased risk of relapse (DFS) and a significantly better survival (MS) when compared to those in the top and middle terciles (see Figures 1 and 2).

In another embodiment, the expression levels of the BRCA1 gene are determined by measuring the expression of the BRCA1 protein. The determination of the expression levels of the BRCA1 protein can be carried out by immunological techniques such as e.g. ELISA, Western Blot or immunofluorescence. Western blot is based on the detection of proteins previously resolved by gel electrophoreses under denaturing conditions and immobilized on a membrane, generally nitrocellulose by the incubation with an antibody specific and a developing system (e.g. chemoluminiscent). The analysis by immunofluorescence requires the use of an antibody specific for the target protein for the analysis of the expression and subcellular localization by microscopy. Generally, the cells under study are previously fixed with paraformaldehyde and permeabilised with a non-ionic detergent. ELISA is based on the use of antigens or antibodies labelled with enzymes so that the conjugates formed between the target antigen and the labelled antibody results in the formation of enzymatically-active complexes. Since one of the components (the antigen or the labelled antibody) are immobilised on a support, the antibody-antigen complexes are immobilised on the support and thus, it can be detected by the addition of a substrate which is converted by the enzyme to a product which is detectable by, e.g. spectrophotometry or fluorometry. This technique does not allow the exact localisation of the target protein or the determination of its molecular weight but allows a very specific and highly sensitive detection of the target protein in a variety of biological samples (serum, plasma, tissue homogenates, postnuclear supernatants, ascites and the like). In a preferred embodiment, the BRCA1 protein is detected by immunohistochemistry (IHC) analysis using thin sections of the biological sample immobilised on coated slides. The sections are then deparaffinised, if derived from a paraffinised tissue sample, and treated so as to retrieve the antigen. The detection can be carried out in individual samples or in tissue microarrays.

Any antibody or reagent known to bind with high affinity to the target protein can be used for detecting the amount of target protein. It is preferred nevertheless the use of antibody, for example polyclonal sera, hybridoma supernatants or monoclonal antibodies, antibody fragments, Fv, Fab, Fab' y F(ab')2, ScFv, diabodies, triabodies, tetrabodies and humanised antibodies.

In yet another embodiment, the determination of BRCA1 protein expression levels can be carried out by constructing a tissue microarray (TMA) containing the patient samples assembled, and determining the expression levels of BRCA1 protein by immunohistochemistry techniques. Immunostaining intensity can be evaluated by two different pathologists and scored using uniform and clear cut-off criteria, in order to maintain the reproducibility of the method. Discrepancies can be resolved by simultaneous re-evaluation. Briefly, the result of immunostaining can be recorded as negative expression (0) versus positive expression, and low expression (1+) versus moderate (2+) and high (3+) expression, taking into account the expression in tumoral cells and the specific cut-off for each marker. As a general criterion, the cut-offs were selected in order to facilitate reproducibility, and when possible, to translate biological events.

The authors of the present invention have further shown that survival of patients suffering from breast cancer who have been treated with a neoadjuvant therapy based on a combination of an anti-metabolite, an intercalating agent and an alkylating agent also correlates with the expression levels of the progesterone receptor. Thus, measurement of both, BRCA1 expression and progesterone receptor expression, can be used as predictive markers of the clinical outcome of patients suffering from breast cancer who have been treated with a neoadjuvant therapy based on based on a combination of an anti-metabolite, an intercalating agent and an alkylating agent. Therefore, in a particular embodiment of the invention and in order to further improve the survival rate in patients with breast cancer and in order to provide more effective therapeutic options according to the invention, the method further comprises determining progesterone receptor expression, wherein if the progesterone receptor expression is positive when compared with reference values, then the patient is a good candidate for a neoadjuvant therapy based on a combination of an anti-metabolite, an intercalating agent and an alkylating agent. In a particular embodiment, the expression levels of the progesterone receptor are determined by measuring the expression of the progesterone receptor protein. The determination of the expression levels of the progesterone receptor protein can be carried out by any immunological means as described before. In a more particular embodiment, the determination of progesterone receptor protein expression levels is carried out by tissue microarray (TMA) determining the expression levels of progesterone receptor protein by immunohistochemistry techniques. Thus, as an illustrative, non limitative example of determining PR expression, immunohistochemical expression of PR is quantified by immnunohistochemistry techniques by means of quantifying the number of PR-positive nuclei in a sample as described, for example, by Mohsin et al. (Modern Pathology; 2004 17, 1545-1554) wherein a tumor sample showing 10% or more PR-positive nuclei is considered as PR positive.

The authors of the present invention have also found that the degree of lymph node involvement, i.e. lymphatic invasion, can be used as a predictive marker of the clinical outcome of patients suffering from breast cancer who have been treated with a neoadjuvant therapy based on based on a combination of an anti-metabolite, an intercalating agent and an alkylating agent. Therefore, in another embodiment the method of the invention further comprises measuring lymph node involvement, wherein if lymph node involvement is negative then, the patient is a good candidate for a neoadjuvant therapy based on a combination of an anti-metabolite, an intercalating agent and an alkylating agent.

The expression "lymph node involvement" as used herein, is understood as the spread of the tumor cells to the lymph nodes and blood vessels located in the vicinity of the tissue which contains the tumor.

The chemotherapy neoadjuvant agents to be used in the method of this invention will be administered in doses commonly employed clinically. Such doses will be calculated in the normal fashion, for example on body surface area.

Examples of antimetabolite drugs which can be used according to the present invention include 5-fluorouracil, cytarabine, gemcitabine, aminopterin, methotrexate, pemetrexed, raltitrexed, cladribine, clofarabine, fludarabine, mercaptopurine, pentostatin, thioguanine, capecitabine, floxuridine, etc.

Examples of alkylating agents include chlorambucil, chlormethine, cyclophosphamide, ifosphamide, melphalan, carmustine, fotemustine, lomustine, streptozocin, carboplatin, cisplatin, oxaliplatin, satraplatin, busulfan, dacarbazine, procarbazine, temozolomide, thioTEPA, treosulfan, and uramustine.

Examples of intercalating agents include daunorubicin, doxorubicin, epirubicin, idarubicin, mitoxantrone, pixantrone, valrubicin.

In a particular embodiment of the invention, the neoadjuvant chemotherapy administered to said breast cancer patient comprises the administration of the anti-metabolite fluorouracil.

5-FU (fluorouracil) acts in several ways, but principally as a thymidylate synthase inhibitor. Interrupting the action of this enzyme blocks synthesis of the pyrimidine thymidine, which is a nucleotide required for DNA replication. Thymidylate synthase methylates deoxyuracilmonophoshate (dUMP) into deoxythyminemonophosphate (dTMP).
Like many anti-cancer drugs, 5-FU's effects are felt system-wide but fall most heavily upon rapidly dividing cells that make heavy use of their nucleotide synthesis machinery. As a pyrimidine analogue, it is transformed inside the cell into different cytotoxic metabolites which are then incorporated into DNA and RNA, finally inducing cell cycle arrest and apoptosis by inhibiting the cell's ability to synthesize DNA. It is an S-phase specific drug and only active during certain cell cycles. In addition to being incorporated in DNA and RNA, the drug has been shown to inhibit the activity of the exosome complex, an exoribonuclease complex of which the activity is essential for cell survival.

In another particular embodiment of the invention, said intercalating agent is epirubicin.

Epirubicin acts by intercalating DNA strands. Intercalation results in complex formation which inhibits DNA and RNA synthesis. It also triggers DNA cleavage by topoisomerase II, resulting in mechanisms that lead to cell death. Binding to cell membranes and plasma proteins may be involved in the compound's cytotoxic effects. Epirubicin also generates free radicals that cause cell and DNA damage. Epirubicin is favoured over doxorubicin, the most popular anthracycline, in some chemotherapy regimens as it appears to cause fewer side-effects. Epirubicin has a different spatial orientation of the hydroxyl group at the 4' carbon of the sugar, which may account for its faster elimination and reduced toxicity. Epirubicin is primarily used against breast and ovarian cancer, gastric cancer, lung cancer, and lymphomas.

In another particular embodiment of the invention, said alkylating agent is cyclophosphamide.

Cyclophosphamide, also known as cytophosphane, is a nitrogen mustard alkylating agent, from the oxazophorines group. It is a "prodrug"; it is converted in the liver to active forms that have chemotherapeutic activity. Cyclophosphamide is converted by mixed function oxidase enzymes in the liver to active metabolites. The main active metabolite is 4-hydroxycyclophosphamide. 4-hydroxycyclophosphamide exists in equilibrium with its tautomer, aldophosphamide. Most of the aldophosphamide is oxidised by the enzyme aldehyde dehydrogenase (ALDH) to make carboxyphosphamide. A small proportion of aldophosphamide is converted into phosphoramide mustard and acrolein. Acrolein is toxic to the bladder epithelium and can lead to hemorrhagic cystitis. This can be prevented through the use of aggressive hydration and/or Mesna.

Recent clinical studies have shown that cyclophosphamide induce beneficial immunomodulatory effects in the context of adoptive immunotherapy. The main effect of cyclophosphamide is due to its metabolite phosphoramide mustard. This metabolite is only formed in cells which have low levels of ALDH. Phosphoramide mustard forms DNA crosslinks between (interstrand crosslinkages) and within (intrastrand crosslinkages) DNA strands at guanine N-7 positions. This leads to cell death. Cyclophosphamide has relatively little typical chemotherapy toxicity as ALDHs are present in relatively large concentrations in bone marrow stem cells, liver and intestinal epithelium. ALDHs protect these actively proliferating tissues against toxic effects phosphoramide mustard and acrolein by converting aldophosphamide to carboxyphosphamide that does not give rise to the toxic metabolites (phosphoramide mustard and acrolein).

Conventional FEC regimen consists of 5-Fluorouracil 600 mg/m², Epirubicin 60 mg/m², Cyclophosphamide 600 mg/m². However, it is feasible to vary said dose according to patients requirements. For example, the dose of epirubicin can be increased by more than 50 per cent with increased dose intensity between 25 and 70 per cent. Additionally, the dose of cyclophosphamide can be increased by more than 100 per cent without severe increase in toxicity for the patient.

The authors of the present invention have found that BRCA1 expression can be used as a good predictive marker of survival in patients suffering from breast cancer. Thus, in another aspect, the present invention refers to a method for determining the clinical response of a patient suffering from breast cancer to neoadjuvant therapy based on a combination of an anti-metabolite, an intercalating agent and an alkylating agent which comprises determining BRCA1 gene expression levels in a sample from said patient, wherein if expression levels of BRCA1 gene are low when compared with reference values then, it is indicative of a good clinical response of said patient to said therapy.

The prediction of the clinical response can be done by using any endpoint measurements used in oncology and known to the skilled practitioner. Useful endpoint parameters to describe the evolution of a disease include:
- disease-free progression which, as used herein, describes the proportion of patients in complete remission who have had no recurrence of disease during the time period under study.
- objective response, which, as used in the present invention, describes the proportion of treated people in whom a complete or partial response is observed.
- tumor control, which, as used in the present invention, relates to the proportion of treated people in whom complete response, partial response, minor response or stable disease ≥ 6 months is observed.
- disease free survival (DFS) which, as used herein, is defined as the length of time after treatment during which a patient survives with no sign of cancer growth.
- six-month progression free survival or PFS6" rate which, as used herein, relates to the percentage of people wherein free of progression in the first six months after the initiation of the therapy and
- median survival (MS) which, as used herein, relates to the time at which half of the patients enrolled in the study are still alive.

In a particular embodiment, prediction of the clinical response is carried out by measuring disease free survival and median survival.

The term "sample" has been previously defined and can be applied to any type of biological sample from a patient. In a particular embodiment, said sample is a tumour tissue sample or portion thereof. In a more particular embodiment, said tumor tissue sample is a breast tumor tissue sample from a patient suffering from breast cancer or a formalin embedded breast tissue sample. In a preferred embodiment, the sample is a tumor biopsy.

In a particular embodiment, the determination of the expression levels of the BRCA1 gene is carried out by measuring the expression levels of the mRNA encoded by the BRCA1 gene or by measuring the expression levels of the BRCA1 gene product using any of the procedures previously mentioned.

As explained before, determining progesterone receptor expression besides BRCA1 expression can be used as a good predictive marker of DFS and MS. Thus, in a particular embodiment, the method also comprises measuring progesterone receptor expression, wherein if the progesterone receptor expression is positive when compared with reference values then, it is indicative of a good clinical response of said patient to said therapy.

In a particular embodiment, the expression levels of the progesterone receptor are determined by measuring the expression of the progesterone receptor protein. The determination of the expression levels of the progesterone receptor protein can be carried out by any immunological means as described before.

In a more particular embodiment, the determination of progesterone receptor protein expression levels is carried out by tissue microarray (TMA) determining the expression levels of progesterone receptor protein by immunohistochemistry techniques.

In another particular embodiment, the method for predicting the clinical response further comprises measuring lymph node involvement, wherein if lymph node involvement is negative then, it is indicative of a good clinical response of said patient to said therapy.

The chemotherapy neoadjuvant agents to be used in the method of this invention will be administered in doses commonly employed clinically. In a particular embodiment of the invention, the neoadjuvant chemotherapy administered to said breast cancer patient comprises the administration of the anti-metabolite fluorouracil. In another particular embodiment, said intercalating agent is epirubicin. In another particular embodiment said alkylating agent is cyclophosphamide.

The findings of the inventors allow the development of personalised therapies for patients suffering from breast cancer wherein the expression of BRCA1 correlates with the possibility that the patient will respond to a neoadjuvant chemotherapy based on a combination of an anti-metabolite, an intercalating agent and an alkylating agent. Thus, in another aspect, the invention relates to a method for evaluating the predisposition of a patient suffering from breast cancer to respond to a neoadjuvant therapy based on a combination of an anti-metabolite, an intercalating agent and an alkylating agent which comprises determining the expression levels of BRCA1 gene in a sample from said patient, wherein if expression levels of BRCA1 gene are low, then it is indicative of favourable predisposition of said patient to respond to a neoadjuvant therapy based on a combination of an anti-metabolite, an intercalating agent and an alkylating agent.

In a particular embodiment of the invention, such sample is a biopsy sample.

In another particular embodiment, the determination of the expression levels of the BRCA1 gene is carried out by measuring the expression levels of the mRNA encoded by the BRCA1 gene or by measuring the expression levels of the BRCA1 gene product using any of the procedures previously mentioned.

The inventors have shown that positive progesterone receptor expression besides low BRCA1 expression correlates with the possibility that the patient will respond to a neoadjuvant chemotherapy based on a combination of an anti-metabolite, an intercalating agent and an alkylating agent. Thus, in a particular embodiment, the method also comprises measuring progesterone receptor expression as explained before, wherein if the progesterone receptor expression is positive when compared with reference values then, it is indicative of favourable predisposition of said patient to respond to a neoadjuvant therapy based on a combination of an anti-metabolite, an intercalating agent and an alkylating agent.

The progesterone receptor (PR) also known as NR3C3 (nuclear receptor subfamily 3, group C, member 3), is an intracellular steroid receptor that specifically binds progesterone.

In a particular embodiment, the expression levels of the progesterone receptor are determined by measuring the expression of the progesterone receptor protein. The determination of the expression levels of the progesterone receptor protein can be carried out by any immunological means as described before.

In a more particular embodiment, the determination of progesterone receptor protein expression levels is carried out by tissue microarray (TMA) determining the expression levels of progesterone receptor protein by immunohistochemistry techniques.

In another particular embodiment, the method for evaluating the predisposition of a patient suffering from breast cancer to respond to a neoadjuvant therapy based on a combination of an anti-metabolite, an intercalating agent and an alkylating agent further comprises measuring lymph node involvement, wherein if lymph node involvement is negative then, it is indicative of favourable predisposition of said patient to respond to a neoadjuvant therapy based on a combination of an anti-metabolite, an intercalating agent and an alkylating agent.

The chemotherapy neoadjuvant agents to be used in the method of this invention will be administered in doses commonly employed clinically. In a particular embodiment of the invention, the neoadjuvant chemotherapy administered to said breast cancer patient comprises the administration of the anti-metabolite fluorouracil. In another particular embodiment, said intercalating agent to be administered is epirubicin. In another particular embodiment said alkylating agent is cyclophosphamide.

In another aspect, the invention refers to a combination of an anti-metabolite, an intercalating agent and an alkylating agent as a neoadjuvant therapy for the treatment of breast cancer in a patient suffering from breast cancer wherein said patient presents low expression levels of the BRCA1 gene.

For the same reasons as explained above, in a particular embodiment of the invention, said patient further presents progesterone receptor positive expression and in another particular embodiment, said patient further presents negative lymph node involvement.

In another aspect, the invention further refers to a method for classifying patients suffering from breast cancer comprising determining:
i) the expression levels of BRCA1 gene; and
ii) progesterone receptor expression;
iii) classifying the patients in four groups according to the results of step i) and ii) defined as
   - low expression levels of BRCA1 gene and positive progesterone receptor expression;
   - low expression levels of BRCA1 and negative progesterone receptor expression;
   - high expression levels of BRCA1 gene and positive progesterone receptor expression; and
   - high expression levels of BRCA1 and negative progesterone receptor expression.

The following examples are provided as merely illustrative and are not to be construed as limiting the scope of the invention.

### EXAMPLE

### Materials and Methods

Tumor biopsies were obtained from 86 patients with locally advanced breast cancer who were treated with four cycles of neoadjuvant chemotherapy fluorouracil, epirubicin and cyclophosphamide (FEC).

**Table 1: Basal clinical characteristics of the 86 patients.**

| **VARIABLES** | **N (%)** |
|---|---|
| **Age; Median (min-max)** | 54 (31-79) |
| **Clinic tumour size (cm)** | 6 (2.50-12) |
| **Menopause** | |
| **Yes** | 50(58.1) |
| **No** | 36(41.9) |
| **Clinical status** | |
| **IIA** | 2(2.3) |
| **IIB** | 22(25.6) |
| **IIIA** | 23(26.7) |
| **IIIB** | 37(43) |
| **IIIC** | 1(1.2) |
| **IV** | 1(1.2) |
| **Initial treatment** | |
| **Surgery** | 1(1.2) |
| **Pre-surgery clinical chemotherapy** | 85(98.8) |
| **Cycles (median, range)** | 4 (4-10) |
| **Clinical response to pre-surgery clinical CT** | |
| **CR** | 0 |
| **PR** | 66(76.7) |
| **SD** | 18(20.9) |
| **PD** | 2(2.3) |
| **Pathological response to pre-surgery clinical CT** | |
| **CR** | 5(5.8) |
| **PR** | 44(51.2) |
| **SD** | 35(40.7) |
| **PD** | 2(2.3) |
| **Surgery** | |
| **Mastectomy+emptying** | 74(86.1) |
| **Mastectomy+sentry node+emptying** | 4(4.7) |
| **Tumorectomy-Biopsy** | 1(1.2) |
| **Tumorectomy+ emptying** | 7(8.1) |
| **Cycles of pre-surgery ChT (median, range)** | 4(4-10) |
| **Pathological status** | |
| **0** | 1(1.2) |
| **I** | 8(9.3) |
| **IIA** | 13(15.1) |
| **IIB** | 19(22.1) |
| **IIIA** | 22(25.6) |
| **IIIB** | 2(2.3) |
| **IIIC** | 19(22.1) |
| **IV** | 1(1.2) |
| **Tumour size (median, range) cm** | 3 (0-12) |
| **Histology** | |
| **Ca. Ductal *in situ*** | 2(2.3) |
| **Car. Mucin** | 1(1.2) |
| **Ductal dif. scamous** | 1(1.2) |
| **Ductal infiltrate** | 75(87.2) |
| **Lobular Infiltrate** | 5(5.8) |
| **Medular** | 1(1.2) |
| **Grade of differentiation** | |
| **I** | 5(5.8) |
| **II** | 32(37.2) |
| **III** | 30(34.9) |
| **NC** | 19(22.1) |
| **Lymph node involvement** | |
| **Positive** | 69(80.2) |
| **Negative** | 17(19.8) |
| **Number of affected ganglia (median, range)** | 3(0-26) |
| **Number of dried ganglia (median, range)** | 12(0-32) |
| **Estrogens' receptors** | |
| **0-4%** | 35(40.7) |
| **5-50%** | 6(7) |
| **50-100%** | 45(52.4) |
| **Progesterone receptors** | |
| 0-4% | 59(68.6) |
| **5-50%** | 5(5.8) |
| **50-100%** | 22(25.6) |
| **BRCA1 by immunohistochemistry** | |
| **0** | 7(8.2) |
| **1** | 1(32.9) |
| **2** | 34(40) |
| **3** | 16(18.8) |
| **BRCA1(mRNA): N; Median(range)** | N=41; |
| | 16.68 (2.93- |
| | 187.40) |
| **Huntingtin by immunohistochemistry** | |
| **0** | 11(13.3) |
| **1** | 40(48.2) |
| **2** | 25(30.1) |
| **3** | 7(8.4) |
| **Her2 by immunohistochemistry** | |
| **0** | 48(55.8) |
| **1** | 12(14) |
| **2** | 10(11.6) |
| **3** | 16(18.6) |
| **CISHTS (HER2 by CISH)** | |
| **Amplification** | 14(16.7) |
| **Low amplification** | 3(3.6) |
| **No amplification** | 67(79.8) |
| **No valuable** | 2 |
| **Vimentin** | |
| **Negative** | 72(84.7) |
| **Positive** | 11(12.9) |
| **Positive Focal** | 2(2.4) |
| **No valuable** | 1 |
| **Cytokeratin 6/7** | |
| **Negative** | 66(78.6) |
| **Positive** | 11(13.1) |
| **Positive Focal** | 7(8.4) |
| **No valuable** | 2 |
| **Groups** | |
| **Her2 positive** | 11(13.1) |
| **Luminar A** | 45(53.6) |
| **Luminar B** | 6(7.1) |
| **Triple negative** | 22(26.2) |
| **No classification** | 2 |
| **22 patients triple negative** | |
| **CK 6/7 and Vimentin positive** | 6(28.6) |
| **CK 6/7 and Vimentin negative** | 7(33.3) |
| **CK 6/7 and/or Vimentin positive and/or negative** | 8(38.1) |
| **CK 6/7 positive and Vimentin no valuable** | 1 |

| | |
|---|---|
| *CT: chemotherapy; CR: Complete response; PR: Partial response; SD: stable disease; PD: progressive disease.* | |

Estrogen receptor(ER), progesterone receptor (PR), HER2, cytokeratin 6/7, vimentin, Huntingtin interacting protein 1 (HIP1) and BRCA1 expression were examined by tissue microarray. HER2 was also assessed by chromogenic *in situ* hybridization (CISH), and BRCA1 mRNA was analyzed in samples of 41 patients by quantitative PCR.
The BRCA1 gene expression was measured as previously described by Specht K, et al. (2001) (Am. J. Pathol., 158, 419-429 and Krafft AE, et al. (1997) Mol. Diagn. 3, 217-230. After standard tissue sample deparaffinization using xylene and alcohols, samples were lysed in a Tris-chloride, EDTA, sodium dodecyl sulphate (SDS) and proteinase K containing buffer. RNA was then extracted with phenol-chloroform-isoamyl alcohol followed by precipitation with isopropanol in the presence of glycogen and sodium acetate. RNA was resuspended in RNA storage solution (Ambion Inc; Austin TX, USA) and treated with DNAse I to avoid DNA contamination. cDNA was synthesized using M-MLV retrotranscriptase enzyme. Template cDNA was added to Taqman Universal Master Mix (AB; Applied Biosystems, Foster City, CA, USA) in a 12.5-µl reaction with specific primers and probe for each gene. The primer and probe sets were designed using Primer Express 2.0 Software (AB). Quantification of gene expression was performed using the ABI Prism 7900HT Sequence Detection System (AB). Primers and probe for BRCA1 mRNA expression analysis were designed according to the Ref Seq NM_007294 (http://www.ncbi.nlm.nih.gov/LocusLink). Forward primer is located in exon 8 (position 4292 bp to 4317 bp), reverse primer in exon 9 (position 4336 bp to 4360 bp), and probe in the exon 8/9 junction (position 4313 bp to 4333 bp). The PCR product size generated with these primers was 69 bp. The primers and 5'labeled fluorescent reporter dye (6FAM) probe were as follows: β-actin: forward 5' TGA GCG CGG CTA CAG CTT 3', reverse 5' TCC TTA ATG TCA CGC ACG ATT T 3', probe 5' ACC ACC ACG GCC GAG CGG 3'; BRCA1: forward 5'GGC TAT CCT CTC AGA GTG ACA TTT TA 3', reverse 5' GCT TTA TCA GGT TAT GTT GCA TGG T 3', probe 5' CCA CTC AGC AGA GGG 3'. Relative gene expression quantification was calculated according to the comparative Ct method using β-actin as an endogenous control and commercial RNA controls (Stratagene, La Jolla, CA) as calibrators. Final results, were determined as follows: 2^{-(ΔCt sample-ΔCt calibrator)}, where ΔC_{T} values of the calibrator and sample are determined by subtracting the C_{T} value of the target gene from the value of the β-actin gene. In all experiments, only triplicates with a standard deviation (SD) of the Ct value <0.20 were accepted. In addition, for each sample analyzed, a retrotranscriptase minus control was run in the same plate to assure lack of genomic DNA contamination.

**Table 2: Basal clinical characteristics of the 41 patients.**

| **VARIABLES** | | N (%) |
|---|---|---|
| **Age; Median(min-max)** | | 55(31-79) |
| **Tumour clinical size (cm)** | | 6.48(2.50-12) |
| **Menopause** | | |
| | **Yes** | 22(53.7) |
| | **No** | 19(46.3) |
| **Clinical status** | | |
| | **IIA** | 0 |
| | **IIB** | 10(24.4) |
| | **IIIA** | 11 (26.8) |
| | **IIIB** | 20(48.8) |
| | **IIIC** | 0 |
| | **IV** | 0 |
| **Cycles of CT complementary (median, range)** | | 4(0-6) |
| **Clinical response to pre-surgery initial CT** | | |
| | **CR** | 0 |
| | **PR** | 31(75.6) |
| | **SD** | 9(22) |
| | **PD** | 1(2.4) |
| **Pathological response to pre-surgery initial CT** | | |
| | **CR** | 1(2.4) |
| | **PR** | 21(51.2) |
| | **SD** | 18(43.9) |
| | **PD** | 1(2.4) |
| **Surgery** | | |
| | **Mastectomy+emptying** | 37(90.2) |
| | **Mastectomy+sentry node+emptying** | 2(4.9) |
| | **Tumorectomy-Biopsy** | 0 |
| | **Tumorectomy+ emptying** | 2(4.9) |
| **Cycles of pre-surgery CT (median, range)** | | 4(4-10) |
| **Pathological status** | | |
| | **0** 0 | |
| | **I** | 3(7.3) |
| | **IIA** | 4(9.8) |
| | **IIB** | 8(19.5) |
| | **IIIA** | 14(34.1) |
| | **IIIB** | 2(4.9) |
| | **IIIC** | 9(22) |
| | **IV** | 0 |

| **VARIABLES** | | **N (%)** |
|---|---|---|
| **Histology** | | |
| | **Ca. Ductal in situ** | 0 |
| | **Mucin Car.** | 0 |
| | **Ductal dif. scamous** | 1(2.4) |
| | **Ductal infiltrate** | 36(87.8) |
| | **Lobular Infiltrate** | 2(4.9) |
| | **Medular** | 1(2.4) |
| **Grade of differentiation** | | |
| | **I** | 0 |
| | **II** | 14(34.1) |
| | **III** | 20(48.8) |
| | **NC** | 7(17.1) |
| **Lymph node involvement** | | |
| | **Positive** | 35(85.4) |
| | **Negative** | 6(14.6) |
| **Number of affected ganglia (median, range)** | | 6(0-26) |
| **Number of dried ganglia (median, range)** | | 15(0-32) |
| **Estrogens' receptors** | | |
| | **0-4%** | 19(47.5) |
| | **5-50%** | 1(2.5) |
| | **50-100%** | 20(50) |
| **Progesterone receptors** | | |
| | **0-4%** | 27(65.9) |
| | **5-50%** | 3(7.3) |
| | **50-100%** | 11(26.8) |
| **BRCA1 by immunohistochemistry** | | |
| | **0** | 4(10) |
| | **1** | 15(37.5) |
| | **2** | 12(30) |
| | **3** | 3(7.5) |
| **BRCA1(RNA): N; Median(range)** | | 41; 16.68(2.93-187.40) |
| **Huntingtin by immunohistochemistry** | | |
| | **0** | 4(10) |
| | **1** | 21(52.5) |
| | **2** | 12(30) |
| | **3** | 3(7.5) |
| **Her2 by immunohistochemistry** | | |
| | **0** | 22(53.7) |
| | **1** | 8(19.5) |
| | **2** | 3(7.3) |
| | **3** | 8(19.5) |
| **CISHTS (HER2 by CISH)** | | |
| | **Amplification** | 8(20) |
| | **low amplification** | 0 |
| | **No amplificated** | 32(80) |
| **No valuable** | | 1 |

| **VARIABLES** | | **N (%)** |
|---|---|---|
| **Vimentin** | | |
| | **Negative** | 35(87.5) |
| | **Positive** | 3(7.5) |
| | **Focal Positive** | 2(5) |
| **No valuable** | | 1 |
| **Cytokeratin 6/7** | | |
| | **Negative** | 31(75.6) |
| | **Positive** | 6(14.6) |
| | **Focal positive** | 4(9.7) |
| **Groups** | | |
| | **Her2 positive** | 6(15) |
| | **Luminar A** | 20(50) |
| | **Luminar B** | 2(5) |
| | **Triple negative** | 12(30) |
| **No classification** | | 1 |

| | | |
|---|---|---|
| *CT: chemotherapy; CR: Complete response; PR: Partial response; SD: stable disease; PD: progressive disease.* | | |

### Results

Pathological response was attained in 57% of patients. Median disease-free survival (DFS) was 30 months (m) and median survival (MS) was 41 months.

Table 3 shows the relationship between BRCA1 expression by terciles (T1, T2 and T3) and the clinical characteristics of the patients.

**Table 3**

| | | **T1** | **T2** | **T3** | **p** |
|---|---|---|---|---|---|
| **Menopause** | | | | | 0.52 |
| | **0** | 5(26.3) | 8(42.1) | 6(31.6) | |
| | **1** | 9(40.9) | 6(27.3) | 7(31.8) | |
| **Age** | | 59(45-73) | 51(32-79) | 54(31-74) | 0.25 |
| **Tumour size** | | 6(4-10) | 7(2.50-12) | 6(4-11) | 0.60 |
| **Cytokeratin 6/7** | | | | | 0.10 |
| | **Negative** | 11(35.5) | 8(25.8) | 12(38.7) | |
| | **Positive** | 3(30) | 6(60) | 1(10) | |
| **HER2 by CISH** | | | | | 0.43 |
| | **HER2 positive by CISH** | 3(37.5) | 4(50) | 1(12.5) | |
| | **HER2 negative by CISH** | 11(34.4) | 10(31.3) | 11(34.4) | |
| **Estrogens' receptor** | | | | | 0.79 |
| | **Negative** | 7(36.8) | 7(36.8) | 5(26.3) | |
| | **Positive** | 7(31.8) | 7(31.8) | 8(36.4) | |
| **progesterone receptor** | | | | | 0.18 |
| | **Negative** | 10(37) | 11(40.7) | 6(22.2) | |
| | **Positive** | 4(28.6) | 3(21.4) | 7(50) | |
| **Vimentin** | | | | | 0.74 |
| | **Negative** | 13(37.1) | 12(34.3) | 10(28.6) | |
| | **Positive** | 1(20) | 2(40) | 2(40) | |
| **Huntingtin by immunohistoch.** | | | | | 0.71 |
| | **0** | 1(25) | 1(25) | 2(50) | |
| | **1** | 6(28.6) | 9(42.9) | 6(28.6) | |
| | **2** | 5(41.7) | 4(33.3) | 3(25) | |
| | **3** | 1(33.3) | 0 | 2(66.7) | |
| **HIP1** | | | | | 0.64 |
| | **Negative (0+1)** | 7(28) | 10(40) | 8(32) | |
| | **Positive (2+3)** | 6(40) | 4(26.7) | 5(33.3) | |
| **Groups** | | | | | 0.43 |
| | **HER2 positive** | 3(50) | 2(33.3) | 1(16.7) | |
| | **Luminar A** | 7(35) | 5(25) | 8(40) | |
| | **Luminar B** | 0 | 2(100) | 0 | |
| | **Triple negative** | 4(33.3) | 5(41.7) | 3(25) | |
| **Lymph node involvement** | | | | | 0.99 |
| | **Negative** | 2(33.3) | 2(33.3) | 2(33.3) | |
| | **Positive** | 12(34.3) | 12(34.3) | 11 (31.4) | |
| **Pathological response** | | | | | 0.32 |
| | **CR** | 0 | 0 | 1(100) | |
| | **PR** | 10(47.6) | 6(28.6) | 5(23.8) | |
| | **SD** | 4(22.2) | 7(38.9) | 7(38.9) | |
| | **PD** | 0 | 1(100) | 0 | |
| **Pathological response** | | | | | 0.26 |
| | **CR+PR** | 10(45.5) | 6(27.3) | 6(27.3) | |
| | **SD+PD** | 4(21.1) | 8(42.1) | 7(36.8) | |
| **Clinical response** | | | | | 0.56 |
| | **PR** | 12(38.7) | 9(29) | 10(32.3) | |
| | **SD** | 2(22.2) | 4(44.4) | 3(33.3) | |
| | **PD** | 0 | 1(100) | 0 | |

| | | | | | |
|---|---|---|---|---|---|
| *CT: chemotherapy; CR: Complete response; PR: Partial response; SD: stable disease; PD: progressive disease.* | | | | | |

Figure 1 shows a Kaplan-Meier survival plot for DFS. Patients have been divided into three groups according to BRCA1 mRNA expression results (Tercile 1, Tercile 2 and Tercile 3), being the group "Tercile 1" the one in which BRCA1 mRNA expression is lower. Results show that DFS is better in patients suffering breast cancer with low BRCA1 expression (Tercile 1) with have been treated with neoadjuvant FEC therapy than in patients with high BRCA1 expression (Terciles 2 and 3). Figure 1 is a Kaplan-Meier survival plot for median survival (MS). In Figure 2 it is shown that median survival is also better in patients with low BRCA1 expression (Tercile 1).

On the other hand, as it is shown in Figure 3 and Table 4 , Klustal-Wallis test (p=0.522) indicated that there was no significant correlation between BRCA1 protein expression measured by immunohistochemistry and BRCA1 mRNA expression measured by quantitative PCR.

**Table 4**

| | **BRCA1 Immunohistochemistry** | | | | | **BRCA1 Immuno Pos/Neg** | | |
|---|---|---|---|---|---|---|---|---|
| | **0** | **1** | **2** | **3** | **p** | **0+1** | **2+3** | **p** |
| **Median values BRCA1 quantitative** | 28.37 | 15.24 | 19.43 | 30.32 | 0.52 | 15.24 | 20.39 | 0.45 |
| **BRCA1 below median** | 2(10) | 10(50) | 6(30) | 2(10) | 0.40 | 12(60) | 8(40) | 0.21 |
| **BRCA1 above median** | 2(10) | 5(25) | 9(45) | 4(20) | | 7(35) | 13(65) | |
| **BRCA1 quartiles** | | | | | 0.30 | | | 0.12 |
| **Q1** | 0 | 4(40) | 4(40) | 2(20) | | 4(40) | 6(60) | |
| **Q2** | 2(20) | 6(60) | 2(20) | 0 | | 8(80) | 2(20) | |
| **Q3** | 0 | 4(40) | 4(40) | 2(20) | | 4(40) | 6(60) | |
| **Q4** | 2(20) | 1(10) | 5(50) | 2(20) | | 3(30) | 7(70) | |

In the multivariate analysis for DFS and MS (Table 5), it is shown that low levels of BRCA1 mRNA, together with positive PR and negative lymph node involvement predicted significantly lower risk of relapse (DFS), while low levels of BRCA1 mRNA and positive PR were the only variables associated with significantly better survival.

**Table 5: Multivariate DFS and survival analysis**

| | | **DFS** | | **SURVIVAL** | |
|---|---|---|---|---|---|
| | **N** | **HR (95% CI)** | **p** | **HR (95% CI)** | **p** |
| **BRCA1 by terciles** | | | | | |
| **Tercile 1** | 13 | 1 | | 1 | |
| **Tercile 2** | 14 | 8.15(2.43-27.29) | **0.001** | 4.50(1.43-14.16) | **0.01** |
| **Tercile3** | 12 | 4.58(1.41-14.90) | **0.01** | 2.68(0.87-8.30) | 0.09 |
| **HIP1** | | | | | |
| **Negative (0+1)** | 24 | 1.19(0.50-2.84) | 0.70 | 0.65(0.26-1.65) | 0.37 |
| **Positive (2+3)** | 15 | 1 | | 1 | |
| **RE** | | | | | |
| **Negative** | **18** | 1.24(0.42-3.66) | 0.70 | 1.38(0.46-4.11) | 0.56 |
| **Positive** | 21 | 1 | | 1 | |
| **PR** | | | | | |
| **Negative** | 26 | 4.36(1.03-18.51) | **0.05** | 2.43(0.62-9.52) | 0.20 |
| **Positive** | 13 | 1 | | 1 | |
| **HER2 by CISH** | | | | | |
| **Negative** | 31 | 1 | | 1 | |
| **Positive** | 8 | 0.61(0.20-1.89) | 0.40 | 0.84(0.28-2.51) | 0.75 |
| **Lymph node status** | | | | | |
| **Negative** | 6 | 1 | | 1 | |
| **Positive** | 33 | 15.17(1.82-126.15) | **0.01** | 3.19(0.70-14.60) | 0.14 |

**Table 6. Univariate DFS and survival analysis**

| | | **DFS** | | **SURVIVAL** | |
|---|---|---|---|---|---|
| | **N** | **HR (95% CI)** | **p** | **HR (95% CI)** | **p** |
| **BRCA1 by terciles** | | | | | |
| **Tercile 1** | 14 | 1 | | 1 | |
| **Tercile 2** | 14 | 4.55(1.61-12.85) | **0.004** | 3.90(1.34-11.38) | **0.01** |
| **Tercile3** | 13 | 2.42(0.87-6.76) | 0.09 | 2.41(0.82-7.09) | 0.11 |
| **HIP1** | | | | | |
| **0** | 11 | 2.99(0.93-9.67) | 0.07 | 2.34(0.73-7.49) | 0.15 |
| **1** | 40 | 1.05(0.36-3.07) | 0.93 | 0.80(0.27-2.37) | 0.69 |
| **2** | 25 | 1.10(0.36-3.39) | 0.87 | 1.22(0.40-3.70) | 0.73 |
| **3** | 7 | 1 | | 1 | |
| **HIP1** | | | | | |
| **Negative (0+1)** | 51 | 1.23(0.68-2.23) | 0.49 | 0.91(0.50-1.64) | 0.74 |
| **Positive (2+3)** | 32 | 1 | | 1 | |
| **RE** | | | | | |
| **Negative** | 35 | 2.25(1.28-3.95) | **0.005** | 2.51(1.41-4.47) | **0.002** |
| **Positive** | 51 | 1 | | 1 | |
| **PR** | | | | | |
| **Negative** | 59 | 1.40(0.76-2.58) | 0.28 | 1.74(0.90-3.36) | 0.10 |
| **Positive** | 27 | 1 | | 1 | |
| **HER2 by CISH** | | | | | |
| **Negative** | 67 | 1 | | 1 | |
| **Positive** | 17 | 1.38(0.70-2.71) | 0.35 | 1.53(0.78-3.03) | 0.22 |
| **Ganglia status** | | | | | |
| **Negative** | 17 | 1 | | 1 | |
| **Positive** | 69 | 2.25(0.95-5.28) | 0.06 | 2.03(0.86-4.78) | 0.11 |

### Conclusions

The inventors have provided evidences to support a major role for BRCA1 gene expression as a predictive marker of DFS and MS in breast cancer. These findings can be useful for customizing chemotherapy.

## Claims

1. A method for selecting an individual neoadjuvant therapy for a patient suffering from breast cancer which comprises determining the expression levels of BRCA1 gene in a sample from said patient, wherein if the expression levels of BRCA1 gene are low when compared with reference values, then the patient is a good candidate for a neoadjuvant therapy based on a combination of a anti-metabolite, a intercalating agent and an alkylating agent.

2. Method according to claim 1 further comprising determining progesterone receptor expression, wherein if the progesterone receptor expression is positive when compared with reference values, then the patient is a good candidate for a neoadjuvant therapy based on a combination of an anti-metabolite, an intercalating agent and an alkylating agent.

3. Method according to claims 1 or 2 further comprising measuring lymph node involvement, wherein if lymph node involvement is negative, then the patient is a good candidate for a neoadjuvant therapy based on a combination of an anti-metabolite, an intercalating agent and an alkylating agent.

4. Method according to any of claim 1 to 3, wherein the sample is a tumor biopsy.

5. Method according to any of claims 1 to 4, wherein the BRCA1 gene expression levels are determined by measuring the levels of mRNA encoded by the BRCA1 gene.

6. Method according to any of claims 2 to 5 wherein the progesterone receptor expression is determined by immunohistochemistry.

7. Method according to any of claims 1-6, wherein said anti-metabolite is fluorouracil.

8. Method according to any of claims 1-7, wherein said intercalating agent is epirubicin.

9. Method according to any one of claims 1 to 8, wherein said alkylating agent is cyclophosphamide.

10. A method for determining the clinical response of a patient suffering from breast cancer to neoadjuvant therapy based on a combination of an anti-metabolite, an intercalating agent and an alkylating agent which comprises determining BRCA1 gene expression levels in a sample from said patient, wherein if expression levels of BRCA1 gene are low when compared with reference values, then it is indicative of a good clinical response of said patient to said therapy.

11. Method according to claim 10 further comprising determining progesterone receptor expression, wherein if the progesterone receptor expression is positive when compared with reference values, then it is indicative of a good clinical response of said patient to said therapy.

12. Method according to claim 10 or 11 further comprising measuring lymph node involvement, wherein if lymph node involvement is negative, then it is indicative of a good clinical response of said patient to said therapy.

13. Method according to any of claims 10 to 12, wherein the sample is a tumor biopsy.

14. Method according to any of claims 10 to 13, wherein the BRCA1 gene expression levels are determined by measuring the levels of mRNA encoded by the BRCA1 gene.

15. Method according to any of claims 11 to 14 wherein progesterone receptor expression is determined by immunohistochemistry.

16. Method according to claims 10 to 15, wherein said anti-metabolite is fluorouracil.

17. Method according to claims 10 to 16, wherein said intercalating agent is epirubicin.

18. Method according to any one of claims 10 to 17, wherein said alkylating agent is cyclophosphamide.

19. Method according to claims 10 to 18, wherein said clinical response is measured as disease-free survival.

20. Method according to claim 10 to 18, wherein said clinical response is measured as overall survival.

21. Method for evaluating the predisposition of a patient suffering from breast cancer to respond to a neoadjuvant therapy based on a combination of an anti-metabolite, an intercalating agent and an alkylating agent which comprises determining the expression levels of BRCA1 gene in a sample from said patient, wherein if expression levels of BRCA1 gene are low, then it is indicative of favourable predisposition of said patient to respond to a neoadjuvant therapy based on a combination of an anti-metabolite, an intercalating agent and an alkylating agent.

22. Method according to claim 21 further comprising determining progesterone receptor expression, wherein if the progesterone receptor expression is positive when compared with reference values then, of favourable predisposition of said patient to respond to a neoadjuvant therapy based on a combination of an anti-metabolite, an intercalating agent and an alkylating agent.

23. Method according to claim 21 or 22 further comprising measuring lymph node involvement, wherein if lymph node involvement is negative, then it is indicative of favourable predisposition of said patient to respond to a neoadjuvant therapy based on a combination of an anti-metabolite, an intercalating agent and an alkylating agent.

24. Method according to any of claims 21 to 23, wherein the sample is a tumor biopsy.

25. Method according to any of claims 21 to 24, wherein the BRCA1 gene expression levels are determined by measuring the levels of mRNA encoded by the BRCA1 gene.

26. Method according to any of claims 21 to 25, wherein progesterone receptor expression is determined by immunohistochemistry.

27. Method according to any of claims 21 to 26, wherein said anti-metabolite is fluorouracil.

28. Method according to any of claims 21 to 27, wherein said intercalating agent is epirubicin.

29. Method according to any of claims 21 to 28, wherein said alkylating agent is cyclophosphamide.

30. A combination of an anti-metabolite, an intercalating agent and an alkylating agent as a neoadjuvant therapy for the treatment of breast cancer in a patient suffering from breast cancer wherein said patient presents low expression levels of the BRCA1 gene

31. Combination according to claim 30, wherein said patient further presents progesterone receptor positive expression.

32. Combination according to claim 31, wherein said patient further presents negative lymph node involvement.

33. A method for classifying patients suffering from breast cancer comprising determining:
i) the expression levels of BRCA1 gene; and
ii) progesterone receptor expression;
iii) classifying the patients in four groups according to the results of step i) and
ii) defined as
- low expression levels of BRCA1 gene and positive progesterone receptor expression;
- low expression levels of BRCA1 and negative progesterone receptor expression;
- high expression levels of BRCA1 gene and positive progesterone receptor expression; and
- high expression levels of BRCA1 and negative progesterone receptor expression.


SEQUENCE LISTING

<110> PANGAEA BIOTECH, S.A.

<120> BRCA1 mRNA EXPRESSION LEVELS PREDICT SURVIVAL IN BREAST CANCER
PATIENTS TREATED WITH NEOADJUVANT CHEMOTHERAPY

<130> P3668EP00

<160> 6

<170> PatentIn version 3.3

<210> 1
<211> 18
<212> DNA
<213> Artificial

<220>
<223> PCR beta-actin forward primer

<400> 1
tgagcgcggc tacagctt 18

<210> 2
<211> 22
<212> DNA
<213> Artificial

<220>
<223> PCR beta-actin reverse primer

<400> 2
tccttaatgt cacgcacgat tt 22

<210> 3
<211> 18
<212> DNA
<213> Artificial

<220>
<223> 5' labeled fluorescent reporter dye (6FAM) probe for beta-actin

<400> 3
accaccacgg ccgagcgg 18

<210> 4
<211> 26
<212> DNA
<213> Artificial

<220>
<223> PCR BRCA1 forward primer

<400> 4
ggctatcctc tcagagtgac atttta 26

<210>  5
<211>  25
<212>  DNA
<213>  Artificial

<220>
<223>  PCR BRCA1 reverse primer

<400>  5
gctttatcag gttatgttgc atggt                                                25


<210>  6
<211>  15
<212>  DNA
<213>  Artificial

<220>
<223>  5' labeled fluorescent reporter dye (6FAM) probe for BRCA1

<400>  6
ccactcagca gaggg                                                           15

SEQUENCE LISTING

<110>  PANGAEA BIOTECH, S.A.

<120>  BRCA1 mRNA EXPRESSION LEVELS PREDICT SURVIVAL IN BREAST CANCER
       PATIENTS TREATED WITH NEOADJUVANT CHEMOTHERAPY

<130>  P3668EP00

<160>  6

<170>  PatentIn version 3.3

<210>  1
<211>  18
<212>  DNA
<213>  Artificial

<220>
<223>  PCR beta-actin forward primer

<400>  1
tgagcgcggc tacagctt                                                    18

<210>  2
<211>  22
<212>  DNA
<213>  Artificial

<220>
<223>  PCR beta-actin reverse primer

<400>  2
tccttaatgt cacgcacgat tt                                               22

<210>  3
<211>  18
<212>  DNA
<213>  Artificial

<220>
<223>  5' labeled fluorescent reporter dye (6FAM) probe for beta-actin

<400>  3
accaccacgg ccgagcgg                                                    18

<210>  4
<211>  26
<212>  DNA
<213>  Artificial

<220>
<223>  PCR BRCA1 forward primer

<400>  4
ggctatcctc tcagagtgac atttta                                           26

<210>  5
<211>  25
<212>  DNA
<213>  Artificial

<220>
<223>  PCR BRCA1 reverse primer

<400>  5
gctttatcag gttatgttgc atggt                                          25


<210>  6
<211>  15
<212>  DNA
<213>  Artificial

<220>
<223>  5' labeled fluorescent reporter dye (6FAM) probe for BRCA1

<400>  6
ccactcagca gaggg                                                     15